# EUROPÄISCHE PATENTSCHRIFT

(11) **EP 0 876 141 B1**
(45) Veröffentlichungstag und Bekanntmachung des Hinweises auf die Patenterteilung: **29.08.2001**
(21) Anmeldenummer: 97901537.7
(22) Anmeldetag: 17.01.1997
(51) Int. Cl.: A61K 9/20

(54) **ZUBEREITUNGEN NICHTSTEROIDALER ANALGETIKA**
PREPARATIONS OF NON-STEROIDAL ANALGESICS
PREPARATIONS D'ANALGESIQUES NON STEROIDIENS

(30) Priorität: 23.01.1996 DE 19602206
(43) Veröffentlichungstag der Anmeldung: 11.11.1998
(73) Patentinhaber: BASF AKTIENGESELLSCHAFT, 67056 Ludwigshafen (DE)
(72) Erfinder: BREITENBACH, Jörg, D-68199 Mannheim (DE); ROSENBERG, Joerg, D-67158 Ellerstadt (DE)
(86) Internationale Anmeldenummer: EP9700185
(87) Internationale Veröffentlichungsnummer: WO9726866

(56) Entgegenhaltungen:
- EP-A- 0 240 904
- EP-A- 0 686 392
- WO-A-96/29053
- WO-A-96/29060
- WO-A-96/29061

## Beschreibung

Die vorliegende Erfindung betrifft Zubereitungen nichtsteroidaler Analgetika mit antipyretischer und antiphlogistischer Wirkung, erhältlich durch Extrusion einer Schmelze, enthaltend neben einem oder mehreren Wirkstoffen eine Mischung
a) 40 bis 99,5 Gew.-% Homopolymeren des N-Vinylpyrrolidons mit einem K-Wert nach Fikentscher von 30,
b) 0,25 bis 59,75 Gew.-% an wasserlöslichen N-Vinylcopolymeren
c) 0,25 bis 10 Gew.-% an einem oder mehreren physiologisch akzeptablen Salzen des Natriums oder des Kaliums,
wobei die Mengenangaben sich auf die Summe von a), b) und c) beziehen, und anschließender Formgebung.

Weiterhin betrifft die Erfindung ein Verfahren zur Herstellung solcher Zubereitungen.

Gerade bei Analgetika ist die schnelle Freisetzung des Wirkstoffs zur Erzielung einer rasch einsetzenden schmerzstillenden Wirkung von entscheidender Bedeutung.

Im Falle von schlecht wasserlöslichen Wirkstoffen wie sie beispielsweise die analgetisch wirksamen organischen Säuren darstellen, ist eine schnelle Freisetzung ausreichender Dosen oft nicht einfach zu erreichen (vgl. Deutsche Apotheker Zeitung, Nr. 32, Seite 54).

In der EP-A 607 467 wird vorgeschlagen, die schnelle Freisetzung von Ibuprofen durch einen Zusatz von basischen Salzen zu fördern, welche während des Pelletiervorgangs in Form wäßriger Lösungen auf den mit einem Hilfsstoff vorgemischten Wirkstoff aufgebracht werden. Die Pellets werden anschließend auf herkömmliche Weise zu Tabletten verpreßt. Diese Vorgehensweise ist jedoch relativ aufwendig und deshalb ökonomisch wenig günstig.

Nach neueren Untersuchungen läßt sich eine schnelle Freisetzung von Ibuprofen durch Einsatz der entsprechenden Lyrinsalze erzielen (G. Geisslinger etal., Drug. Invest. SC4), 238-242, 1993).

Es ist weiterhin bekannt, daß durch die Extrusion wirkstoffhaltiger Polymerschmelzen mit anschließender kontinuierlicher Formgebung die Herstellung von Arzneiformen auf sehr wirtschaftliche Weise erfolgen kann.

In der EP-B 240 904 wird ein solches Verfahren zur Herstellung fester pharmazeutischer Formen durch Extrusion wirkstoffhaltiger Polymerschmelzen beschrieben, wobei als Polymere Homo- oder Copolymere des N-Vinylpyrrolidons verwendet werden.

Bei einem solchen Verfahren besteht jedoch das grundsätzliche Problem, daß die matrixbildenden Polymere zum einen bei den Verarbeitungstemperaturen ausreichend thermoplastisch verarbeitbar sind bzw. durch Zusatz einer weichmachenden Substanz verarbeitbar werden, zum anderen aber unter den üblichen Lagerungsbedingungen zu stabilen Arzneiformen führen, bei denen kein "kalter Fluß" eintritt.

Dieses Problem ist umso schwerer lösbar, wenn man schnell freisetzende Arzneiformen herstellen will. Dazu eignen sich normalerweise vor allem relativ niedermolekulare Polymere, die sich in Verdauungssäften schnell auflösen. Gerade diese zeigen aber verstärkt das Phänomen des "kalten Flusses" bei den fertigen Arzneiformen. Höhermolekulare Polymere sind üblicherweise nicht schnell freisetzend und können ohne Weichmacher kaum extrudiert werden, da die Glastemperatur (DIN 52324) wesentlich höher liegt.

Ein zusätzliches Problem stellt sich, wenn man transparente Arzneiformen durch Schmelzextrusion herstellen will.

Aufgabe der vorliegenden Erfindung war es transparente, schnell freisetzende Zubereitungen nichtsteroidaler Analgetika zu finden, die auf einfache Weise durch Schmelzextrusion mit anschließender Formgebung herstellbar sind und eine gute Lagerstabilität aufweisen.

Demgemäß wurden die eingangs definierten Zubereitungen gefunden.

Als Wirkstoffe kommen erfindungsgemäß nichtsteroidale Analgetika mit antipyretischer und antiphlogistischer Wirkung in Betracht, wie sie auch für die symptomatische antirheumatische Therapie eingesetzt werden.

Geeignete Wirkstoffe sind demgemäß Derivate der Salicylsäure wie Acetylsalicylsäure sowie Derivate anderer organischer Säuren und Pyrazolderivate und, soweit vorhanden, deren physiologisch verträgliche Salze. So kommen als Wirkstoffe Arylsäurederivate wie Diclofenac, Tolmetin oder Zomepirac in Betracht, weiterhin Arylpropylsäurederivate wie Ibuprofen, wobei sowohl enantiomerenreines S(+)-Ibuprofen als auch ein mit diesem Enantiomeren angereichertes Racemat umfaßt ist, ebenso wie D,L-Lysinsalze des Ibuprofens, Naproxen, Fenoprofen, Flurbiprofen oder Ketoprofen oder auch Indol- und Indenessigsäurederivate wie beispielsweise Indometacin oder Sulindac. Geeignete Pyrazolderivate sind beispielsweise Phenazon, Aminophenazon, Metamizol, Propyphenazon, Phenylbutazon oder Oxyphenbutazon.

Bevorzugte Wirkstoffe sind Ibuprofen, Acetylsalicylsäure und Ketoprofen, Sulindac, Indometacin, Flurbiprofen.

Es können auch Wirkstoffgemische eingesetzt werden. Weiterhin kommen auch Mischungen der Analgetika mit Koffein oder Kodein in Betracht.

Als Komponenten a) enthalten die erfindungsgemäßen Zubereitungen ein Homopolymeres des N-Vinylpyrrolidons mit einem K-Wert nach Fikentscher von 30. Dieses Homopolymere ist gut thermo- und wasserlöslich, wobei "wasserlöslich" bedeutet, daß sich in 100 g Wasser bei 20°C mindestens 0,5 g, vorzugsweise mindestens 2 g des Polymeren lösen, gegebenenfalls auch kolloidal. Die Herstellung des Homopolymeren ist allgemein bekannt.

Als Komponenten b) kommen wasserlösliche Copolymerisate des N-Vinylpyrrolidons in Betracht. Geeignete Copolymerisate sind vor allem solche mit Vinylacetat in Anteilen von 10 - 50 %, besonders bevorzugt solche, die durch Copolymerisation von 60 Gew.-% N-Vinylpyrrolidon und 40 Gew.-% Vinylacetat erhalten werden.

Als Komponenten c) kommen physiologisch verträgliche Natrium- und/oder Kaliumsalze auch in Form ihrer Hydrate in Betracht, beispielsweise Natriumacetat, Kaliumacetat, Natriumcarbonat, Kaliumcarbonat, Dinatriumhydrogencarbonat, Kaliumhydroxid, Natriumchlorid oder Kaliumchlorid, Natriumtricitrat wobei Natriumacetat bevorzugt ist, besonders bevorzugt als Natriumacetattrihydrat.

Die Mengenverhältnisse der Komponenten a), b) und c) werden erfindungsgemäß so gewählt, daß die Zubereitungen ein Mischung aus
a) 40 bis 99,5 Gew.-%, bevorzugt, 45 bis 95 Gew.-% der Komponente a),
b) 0,25 bis 59,75 Gew.-%, bevorzugt 0,5 bis 50 Gew.-%, der Komponente b), und
c) 0,25 bis 10 Gew.-%, bevorzugt 0,5 bis 7 Gew.-% der Komponente c) enthalten,
   wobei die Mengenangabe sich auf die Summe von a), b) und c) beziehen.

Ganz besonders bevorzugt sind Arzneiformen, enthaltend neben dem Arzneistoff eine Mischung aus
a) 60 bis 85 Gew.-% der Komponente a)
b) 5 bis 35 Gew.-% der Komponente b)
c) 0,5 bis 5 Gew.-% der Komponente c).

Der Mengenanteil der Summe der Komponenten a), b), c) an der Arzneiform beträgt vorzugsweise 60 bis 85 Gew.-%. Dementsprechend enthalten die Arzneiformen vorzugsweise 15 bis 40 Gew.-% eines oder mehrerer Wirkstoffe.

Weiterhin können die Arzneiformen 0 bis 5 Gew.-% weiterer üblicher Hilfsstoffe in den üblichen Mengen enthalten.

Das Mischen des Wirkstoffs oder der Wirkstoffe mit den polymeren Bindemitteln und gegebenenfalls galenischen Zusätzen kann vor oder nach dem Schmelzen des polymeren Bindemittels nach den in der Technik üblichen Verfahren erfolgen. Bevorzugt wird das Mischen im Extruder, vorzugsweise einem Zweischneckenextruder oder einem Einschneckenextruder mit Mischabteil.

Die Schmelzen sind lösungsmittelfrei. Damit ist gemeint, daß kein Wasser und kein organisches Lösungsmittel zugesetzt wird.

Die Herstellung erfolgt durch Extrusion bei 50 bis 180°C, vorzugsweise 60 bis 150°C und anschließende Verformung des noch plastischen Stranges, z.B. durch Verformen zu Tabletten, beispielsweise gemäß EP-A 240 906 durch Hindurchführen des Stranges zwischen zwei gegenläufig angetriebenen Walzen mit einander gegenüberliegenden Vertiefungen im Walzenmantel, deren Ausführung die Tablettenform bestimmt. Auch Kaltabschlag kommt in Betracht.

Bevorzugt wird der sogenannte Heißabschlag. Dabei werden die Stränge unmittelbar nach dem Austritt aus der Düsenanordnung am Extruder durch beispielsweise rotierende Messer oder eine andere geeignete Anordnung zerkleinert, zweckmäßig in Stücke, deren Länge etwa gleich dem Strangdurchmesser ist. Diese abgeschlagenen Schmelzteilchen kühlen im Luft- oder Gasstrom so weit ab, daß die Oberfläche vor einer Berührung mit anderen Teilchen oder einer Gefäßwand bereits klebfrei ist, andererseits die Teilchen aber noch so plastisch sind, daß sie durch Zusammenstöße, z.B. mit der Wandung eines angeschlossenen Cyclons, eine sphärische Form erhalten. Man erhält so in einfacher Weise weitgehend kugel- oder linsenförmige Teilchen mit Durchmessern von 0,5 bis 4, vorzugsweise 0,8 bis 2 mm. Die bevorzugten kleineren Teilchen sind in erster Linie zum Füllen von Kapseln geeignet, können aber auch anschließend unter Zusatz weiterer Hilfsstoffe zu Tabletten verpreßt werden.
Die festen Arzneiformen können auch mit einem üblichen Überzug zur Verbesserung des Aussehens und/oder des Geschmacks (Dragee) versehen werden.

Die erfindungsgemäßen Zubereitungen von nichtsteroidalen Analgetika mit antipyretischer und antiphlogistischer Wirkung sind transparent, lagerstabil und schnell freisetzend. "Schnell freisetzend" bedeutet, daß die Freisetzung des Wirkstoffs nach 30 min, gemessen nach der Paddle-Methode nach USP XXII, mindestens 70 % beträgt.

Überraschenderweise waren trotz Verwendung eines relativ hochmolekularen Polymers sogar Arzneiformen mit hohen Gewichten wie 1000 mg schnell freisetzend. Vorteilhaft an großen Tabletten ist auch, daß sie als Lutschtabletten angewendet werden können, ohne verschluckt zu werden. Gerade bei älteren Patienten oder bei Patienten mit Dysphagie ist das Schlucken größerer Tabletten of mit Schwierigkeiten verbunden, so daß schnell freisetzende Lutschtabletten große Vorzüge aufweisen.

Überraschend ist, daß für jede Formgröße (1000, 850 oder 650 mg Bolus) eine Mischung der Komponenten a und b gefunden werden kann, die eine maximale Freisetzung erreicht, welche deutlich über den Freisetzungen von Mischungen anderer Zusammensetzung, oder insbesondere der einzelnen Polymere alleine liegt.

Auch in der Literatur beschriebene, durch Solvensprozeß hergestellte, feste Lösungen, werden in der Freisetzungsrate übertroffen (M. Najib, M. Suleilman, A. Malakh, 32 (1986) 229-236.)

### Beispiele

Die in den Beispielen jeweils angegebenen Zusammensetzungen wurden vorgemischt und in den Einzug eines Doppelschnecken-Extruders (Werner & Pfleiderer, ZSK 30) eingetragen. Die Schmelzextrusion erfolgte mit einem Produktdurchsatz von 3 bis 4 kg/h. Die Temperaturen der einzelnen Temperaturzonen ("Schüsse") des Extruders sowie die Temperatur der beheizten Düsenleiste ist bei den Versuchen jeweils angegeben. Aus dem Extrudat wurden gemäß dem in der EP-B 240 906 beschriebenen Kalandrierverfahren Tabletten mit einem Gewicht von 1000, 850 und 650 mg hergestellt.

### Extrusionsbedingungen:

| | |
|---|---|
| Schuß 1 | 43°C |
| Schuß 2 | 57°C |
| Schuß 3 | 120°C |
| Schuß 4 | 100°C |
| Schuß 5 | 100°C |
| Kopf | 100°C |
| Düse | 100°C |
| Kalandertemperatur | 18°C |

Die Wirkstofffreisetzung wurde mittels der Paddle-Methode nach USP XXII, US-Arzneibuch gemessen. Diese in vitro-Prüfungsmethode dient zur Bestimmung der Lösungsrate von wirkstoffhaltigen Formlingen, z.B. Tabletten.
Hierzu wurden 900 ml eines Phosphatpuffers mit einem pH-Wert von 7,2 in einem 1 1-Gefäß mit Rundboden auf 37°C temperiert. Eine entsprechende Menge an Arzneiform wurde eingewogen. Die Wirkstofffreisetzung der Tabletten wurde in diesem No-Change-Test nach USP XXII bei einer Paddle-Drehzahl von 150 Upm nach jeweils 30 Min. UV-spektroskopisch bestimmt.

### Beispiel 1

### Tablettengewicht 850 mg

| Zusammensetzung: | |
|---|---|
| Ibuprofen | 26,0 Gew.-% |
| Kollidon® K 30¹⁾ | 56,5 Gew.-% |
| Kollidon® VA 64²⁾ | 15,0 Gew.-% |
| Na-Acetat x 3 H₂O | 2,0 Gew.-% |
| feinteilige Kieselsäure | 0,5 Gew.-% |
| Freisetzung nach 30 min: | 88,0 Gew.-% |

| | |
|---|---|
| ¹⁾ PVP-Homopolymer, K-Wert nach Fikentscher von 30 | |
| ²⁾ Copolymer, erhalten aus 60 Gew.-% N-Vinylpyrrolidon und 40 Gew.-% Vinylacetat, K-Wert 30. | |

### Beispiel 2

### Tablettengewicht 650 mg

| Zusammensetzung: | |
|---|---|
| Ibuprofen | 33,0 Gew.-% |
| Kollidon® K 30 | 44,2 Gew.- % |
| Kollidon® VA 64 | 20,0 Gew.-% |
| Na-Acetat x 3 H₂O | 2,0 Gew.-% |
| feinteilige Kieselsäure | 0,8 Gew.-% |
| Freisetzung nach 30 min: | 90,0 Gew.-% |

### Beispiel 3

### Tablettengewicht 850 mg

| Zusammensetzung: | |
|---|---|
| Ibuprofen | 23,5 Gew.-% |
| Kollidon® K 30 | 59,0 Gew.-% |
| Kollidon® VA 64 | 15,0 Gew.-% |
| Na-Acetat x 3 H₂O | 2,0 Gew.-% |
| feinteilige Kieselsäure | 0,5 Gew.-% |

Es wurde die Bioverfügbarkeit beim Hund bestimmt:
6 Hunde, Einmalapplikation von 1 Tablette pro Tier
Crossover-Studie mit 1 Woche wash-out

Die Wirkstoffgehalte im Plasma wurden durch HPLC mit UV-Detektion bestimmt.

Zum Vergleich wurde unter den gleichen Bedingungen ein handelsübliches Päparat, enthaltend 342 mg Ibuprofen-D,L-Lysinat (entspricht 200 mg Ibuprofen) verabreicht.

Die Ergebnisse sind in der Tabelle aufgelistet.

**Tabelle:**

| Tabelle: Pharmakokinetik Hund | | |
|---|---|---|
| | Plasmaspiegel [µg/ml] x 10³ | |
| Zeit [h] | Tablette gemäß Bsp. 3 | Ibuprofen-Lysinat |
| 0,25 | 14,98 | 6,37 |
| 0,5 | 35,12 | 13,45 |
| 0,75 | 42,19 | 25,83 |
| 1,0 | 51,06 | 35,27 |
| 1,5 | 55,16 | 46,07 |
| 2,0 | 49,13 | 47,82 |
| 2,5 | 43,23 | 40,65 |
| 3,0 | 38,12 | 39,66 |
| 4,0 | 34,63 | 32,42 |
| 6,0 | 23,49 | 35,72 |
| 8,0 | 14,40 | 14,46 |
| 24,0 | 0,71 | 0,90 |

## Patentansprüche

1. Zubereitungen nichtsteroidaler Analgetika mit antipyretischer und antiphlogistischer Wirkung, erhältlich durch Extrusion und Formgebung einer Schmelze, enthaltend neben einem oder mehreren Wirkstoffen eine Mischung aus
a) 40 bis 99,5 Gew.-% eines Homopolymeren des N-Vinylpyrrolidons mit einem K-Wert nach Fikentscher von 30,
b) 0,25 bis 59,75 Gew.-% eines wasserlöslichen Copolymeren des N-Vinylpyrrolidons, und
c) 0,25 bis 10 Gew.-% eines oder mehrerer physiologisch akzeptabler Salze des Natriums oder des Kaliums,
wobei sich die Mengenangaben auf die Summe der Komponenten a), b) und c) beziehen.

2. Zubereitungen nach Anspruch 1, enthaltend 15 bis 40 Gew.-% eines oder mehrerer Wirkstoffe und 60 bis 85 Gew.-% einer Mischung der Komponenten a), b) und c).

3. Zubereitungen nach Anspruch 1 oder 2, enthaltend eine Komponente b), welche durch Copolymerisation von 60 Gew.-% N-Vinylpyrrolidon und 40 Gew.-% Vinylacetat erhalten wird.

4. Zubereitungen nach einem der Ansprüche 1 bis 3, enthaltend als Komponente c) Natriumacetat.

5. Zubereitungen nach einem der Ansprüche 1 bis 4, enthaltend als Wirkstoff Ibuprofen.

6. Verfahren zur Herstellung von Zubereitungen gemäß einem der Ansprüche 1 bis 5, **dadurch gekennzeichnet**, daß man eine Schmelze, enthaltend einen oder mehrere Wirkstoffe und eine Mischung aus
a) 40 bis 99,5 Gew.-% eines Homopolymeren des N-Vinylpyrrolidons mit einem K-Wert nach Fikentscher von 30,
b) 0,25 bis 59,75 Gew.-% eines wasserlöslichen Copolymeren des N-Vinylpyrrolidons, und
c) 0,25 bis 10 Gew.-% eines oder mehrerer physiologisch akzeptabler Salze des Natriums oder des Kaliums,
wobei sich die Mengenangaben auf die Summe der Komponenten a), b) und c) beziehen, extrudiert und die noch plastische Schmelze verformt.

## Claims

1. A preparation of a non-steroidal analgesic with antipyretic and antiinflammatory effect, obtainable by extrusion and shaping of a melt, comprising, besides one or more active ingredients, a mixture of
a) 40-99.5% by weight of a homopolymer of N-vinylpyrrolidone with a Fikentscher K value of 30,
b) 0.25-59.75% by weight of a water-soluble copolymer of N-vinylpyrrolidone, and
c) 0.25-10% by weight of one or more physiologically acceptable salts of sodium or potassium,
where the stated amounts are based on the total of components a), b) and c).

2. A preparation as claimed in claim 1, comprising 15-40% by weight of one or more active ingredients and 60-85% by weight of a mixture of components a), b) and c).

3. A preparation as claimed in claim 1 or 2, comprising a component b) which is obtained by copolymerization of 60% by weight of N-vinylpyrrolidone and 40% by weight of vinyl acetate.

4. A preparation as claimed in any of claims 1 to 3, comprising sodium acetate as component c).

5. A preparation as claimed in any of claims 1 to 4, comprising ibuprofen as active ingredient.

6. A process for producing a preparation as claimed in any of claims 1 to 5, which comprises extruding a melt comprising one or more active ingredients and a mixture of
a) 40-99.5% by weight of a homopolymer of N-vinylpyrrolidone with a Fikentscher K value of 30,
b) 0.25-59.75% by weight of a water-soluble copolymer of N-vinylpyrrolidone, and
c) 0.25-10% by weight of one or more physiologically acceptable salts of sodium or potassium,
where the stated amounts are based on the total of components a), b) and c), and shaping the melt while still plastic.

## Revendications

1. Préparations d'analgésiques non-stéroïdiens ayant une activité antipyrétique et antiphlogistique, pouvant être obtenue par extrusion et mise en forme d'une masse fondue contenant, outre une ou plusieurs substances actives, un mélange de
a) 40 à 99,5 % en poids d'un homopolymère de N-vinylpyrrolidone ayant un indice K selon Fikentscher de 30,
b) 0,25 à 59,75 % en poids d'un copolymère hydrosoluble de N-vinylpyrrolidone, et
c) 0,25 à 10 % en poids d'un ou plusieurs sels physiologiquement acceptables de sodium ou de potassium,
tandis que les indications de quantité se rapportent à la somme des composants a), b) et c).

2. Préparations selon la revendication 1, contenant 15 à 40 % en poids d'une ou plusieurs substances actives et 60 à 85 % en poids d'un mélange des composants a), b) et c).

3. Préparations selon la revendication 1 ou 2, contenant un composant b), qui est obtenu par copolymérisation de 60 % en poids de N-vinylpyrrolidone et 40 % en poids d'acétate de vinyle.

4. Préparations selon l'une des revendications 1 à 3, contenant de l'acétate de sodium comme composant c).

5. Préparations selon l'une des revendications 1 à 4, contenant de l'Ibuprofène comme substance active.

6. Procédé pour la production de préparations selon l'une des revendications 1 à 5, caractérisé par le fait qu'on extrude une masse fondue, contenant une ou plusieurs substances actives et un mélange de
a) 40 à 99,5 % en poids d'un homopolymère de N-vinylpyrrolidone ayant un indice K selon Fikentscher de 30,
b) 0,25 à 59,75 % en poids d'un copolymère hydrosoluble de N-vinylpyrrolidone, et
c) 0,25 à 10 % en poids d'un ou plusieurs sels physiologiquement acceptables de sodium ou de potassium,
les indications de quantité se rapportant à la somme des composants a), b) et c),
et on met en forme la masse fondue encore plastique.
